Europäisches Patentamt

**European Patent Office**

Office européen des brevets

(19)

(11) Publication number: **0 525 828 A2**

# EUROPEAN PATENT APPLICATION

(12)

(21) Application number: **92116879.5**

(22) Date of filing: **19.01.87**

(51) Int. Cl.5: **C12N 15/49**, C07K 15/04,
C12N 1/21, G01N 33/569

This application was filed on 02 - 10 - 1992 as a divisional application to the application mentioned under INID code 60.

(30) Priority: **01.08.86 US 892680**

(43) Date of publication of application:
**03.02.93 Bulletin 93/05**

(60) Publication number of the earlier application in accordance with Art.76 EPC: **0 255 190**

(84) Designated Contracting States:
**BE CH DE ES FR GB GR IT LI NL SE**

(71) Applicant: **REPLIGEN CORPORATION
One Kendall Square Building 700
Cambridge, MA 02139(US)**

(72) Inventor: **Putney, Scott D.
102 Gloucester Street
Arlington, Massachusetts 02174(US)**
Inventor: **Lynn, Debra
11 Allen Street, Apt. 11
Arlington, Massachusetts 02174(US)**
Inventor: **Javaherian, Kashayar
27 Webster Road
Lexington, Massachusetts 02173(US)**
Inventor: **Mueller, William T.
26 Copeland Street
Watertown, Massachusetts 02172(US)**
Inventor: **Farley, John
261 Culver Riadm No. 9
Rochester, New York 14607(US)**

(74) Representative: **Perry, Robert Edward
GILL JENNINGS & EVERY, Broadgate House,
7 Eldon Street
London EC2M 7LH(GB)**

(54) Recombinant polypeptides and their uses, including assay for aids virus.

(57) A novel 26 kD fusion protein designated protein PB1 is a recombinant HIV envelope protein that is useful in the diagnosis, propylaxis or therapy of AIDS.

EP 0 525 828 A2

This invention relates to recombinant polypeptides and their uses, including assay for HIV (sometimes described as HTLV-III).

HIV displays tropism for the OKT4$^+$ lymphocyte subset; see Klatzmann et al, Science 225 (1984) 59-63.

Antibodies against HIV proteins in the sera of most AIDS and AIDS-related complex (ARC) patients, and in asymptomatic people infected with the virus (Sarngadharan et al, Science 224 (1984) 506-508) have made possible the development of immunologically-based tests that detect antibodies to these antigens. These tests are used to limit the spread of HIV through blood transfusion, by identifying blood samples of people infected with the virus. Diagnostic tests currently available commercially use the proteins of inactivated virus as antigens.

In addition to allowing new approaches for diagnosis, recombinant DNA holds great promise for the development of vaccines against both bacteria and viruses; see Wilson, Bio/Technology 2 (1984) 29-39.

The most widely employed organisms to express recombinant vaccines have been E. coli, S. cerevisiae and cultured mammalian cells. For example, subunit vaccines against foot and mouth disease and malaria have been synthesised in E. coli; see, respectively, Kleid et al, Science 214 (1981) 1125-1129, and Young et al, Science 228 (1985) 958-962. Other examples are hepatitis B surface antigen produced in yeast, and a herpes vaccine produced in mammalian cells; see, respectively, McAleer et al, Nature 307 (1984) 178-180, and Berman et al, Science 227 (1984) 1490-1492.

There is a real need at this time to develop a vaccine for AIDS. No such vaccine is known to exist.

Summary of the Invention

A novel protein, denoted PB1 herein, is shown in Table 5. This recombinant HIV envelope protein can be used in the diagnosis, propylaxis or therapy of AIDS. Further, fragments thereof can be used to stimulate a lymphocyte proliferative response in HIV-infected humans. This then would stimulate the immune system to respond to HIV in such individuals and, therefore, the envelope protein fragments can provide protection and be of therapeutic value.

The present invention also provides DNA sequences encoding the novel proteins, and assay methods using them. The novel protein is encoded on a novel bacterial plasmid which can be used to transform suitable hosts, for example, E. coli, using standard procedures.

In the accompanying drawings:

Figures 1A and 1B are sequential flow charts of the constructions, from plasmid pBG1, of plasmid pREV2.2 which is used to construct vectors encoding novel proteins;

Figure 2 is a diagram of plasmid pREV2.2 and also of the multiple cloning site; and

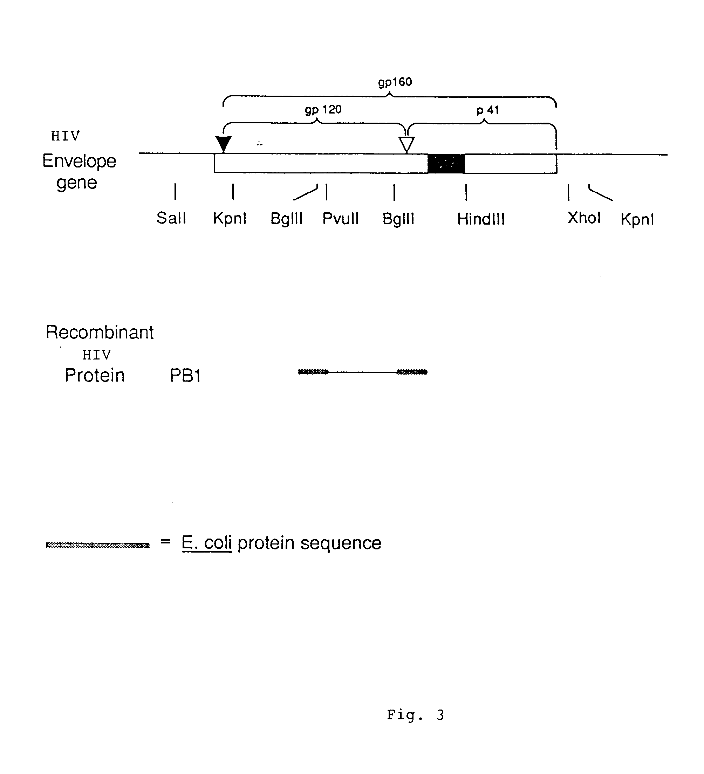

Figure 3 is a schematic representation of the HIV envelope gene and also of the novel recombinant protein obtained therefrom.

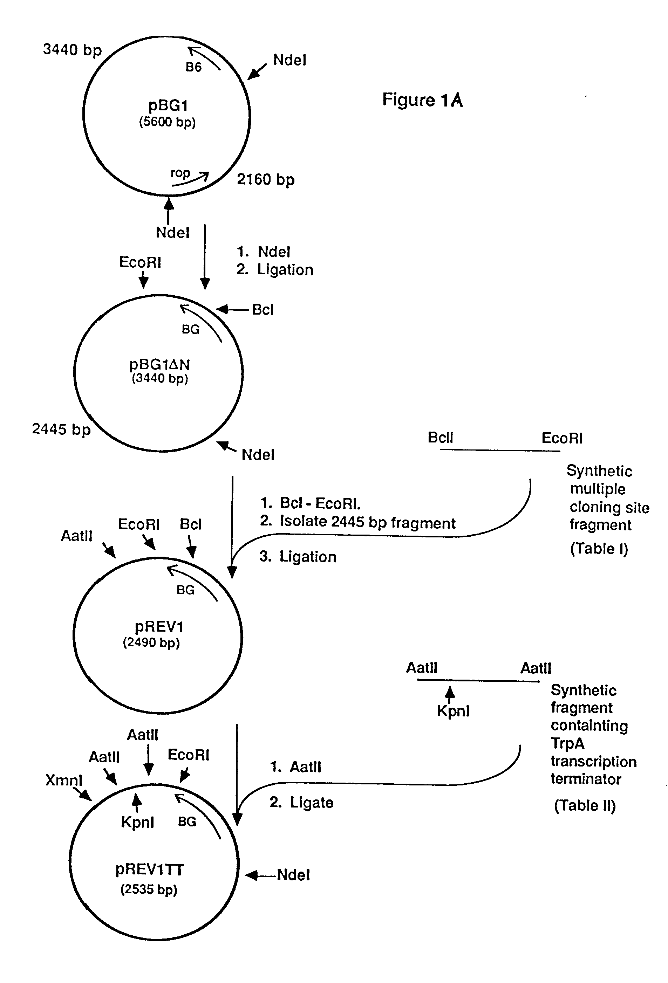

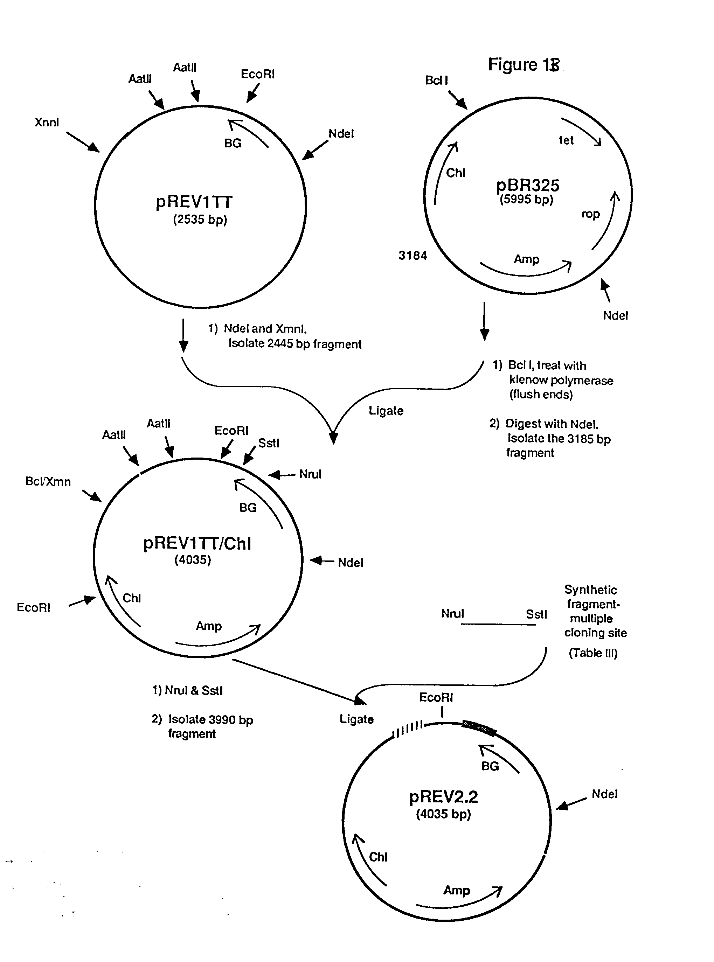

Expression vector plasmid pREV2.2 was constructed from plasmid pBG1 by the route shown in Figure 1 of the drawings. In the product, the hatched region represents TrpA trasc. terminator and the dotted region represents multiple cloning sites for enzymes NruI, MluI, EcoRV, ClaI, BamHI, SalI, HindIII, SmaI, StsI and EcoRI.

Plasmid pPB1 contains approximately 540 base pairs of DNA encoding essentially the HIV env gene from the PvuII site to the BglII site. This plasmid in a suitable host, e.g. E. coli, can be used to produce the novel recombinant HIV 26 kD fusion protein denoted PB1. The amino-acid sequence of fusion protein PB1 is shown in Table 5; the DNA sequence encoding this protein is shown in Table 5A.

The following have been deposited with the Northern Regional Research Laboratory (NRRL), U.S. Department of Agriculture, Peoria, Illinois, USA:

| Culture | Accession No. | Date of Deposit |
|---|---|---|
| E. coli JM103(pREV2.2) | NRRL B-18091 | July 30, 1986 |
| E. coli SG20251(pPB1) | NRRL B-18092 | July 30, 1986 |
| E. coli MS371 (pBG1) | NRRL B-15904 | Nov. 1, 1984 |
| E. coli MS371 | NRRL B-15129 | |
| E. coli SG20251 | NRRL B-15918 | Dec. 12, 1984 |

The novel HTLV-III proteins of the subject invention can be expressed in Saccharomyces cerevisiae using plasmids containing the inducible galactose promoter from this organism (Broach, J.R., Li, Y., Wu, L.C. and Jayaram, M. in Experimental Manipulation of Gene Expression [1983] p. 83, ed. M. Inouye, Academic Press). These plasmids are called YEp51 and YEp52 (Broach, J.R. et al. [1983]) and contain the E. coli origin of replication, the gene for β-lactamase, the yeast LEU2 gene, the 2 μm origin of replication and the 2 μm circle REP3 locus. Recombinant gene expression is driven by the yeast GAL10 gene promoter.

Yeast promoters such as galactose and alcohol dehydrogenase (Bennetzen, J.L. and Hall, B.D. [1982] J. Biol. Chem. 257:3018; Ammerer, G. in Methods in Enzymology [1983] Vol. 101, p. 192), phosphoglycerate kinase (Derynck. R., Hitzeman, R.A., Gray, P.W., Goeddel, D.V., in Experimental Manipulation of Gene Expression [1983] p. 247, ed. M. Inouye, Academic Press), triose phosphate isomerase (Alber, T. and Kawasaki, G. [1982] J. Molec. and Applied Genet. 1:419), or enolase (Innes, M.A. et al. [1985] Science 226:21) can be used.

The genes disclosed herein can be expressed in simian cells. When the genes encoding these proteins are cloned into one of the plasmids as described in Okayama and Berg (Okayama, H. and Berg, P. [1983] Molec. and Cell. Biol. 3:280) and references therein, or COS cells transformed with these plasmids, synthesis of HTLV-III proteins can be detected immunologically.

Other mammalian cell gene expression/protein production systems can be used. Examples of other such systems are the vaccinia virus expression system (Moss, B. [1985] Immunology Today 6:243; Chakrabarti, S., Brechling, K., Moss, B. [1985] Molec. and Cell. Biol. 5:3403) and the vectors derived from murine retroviruses (Mulligan, R.C. in Experimental Manipulation of Gene Expression [1983] p. 155, ed. M. Inouye, Academic Press).

The HTLV-III proteins of the subject invention can be chemically synthesized by solid phase peptide synthetic techinques such as BOC and FMOC (Merrifield, R.B. [1963] J. Amer. Chem. Soc. 85:2149; Chang, C. and Meienhofer, J. [1978] Int. J. Peptide Protein Res. 11:246).

As is well known in the art, the amino acid sequence of a protein is determined by the nucleotide sequence of the DNA. Because of the redundancy of the genetic code, i.e., more than one coding nucleotide triplet (codon) can be used for most of the amino acids used to make proteins, different nucleotide sequences can code for a particular amino acid. Thus, the genetic code can be depicted as follows:

| Phenylalanine (Phe) | TTK | Histidine (His) | CAK |
| Leucine (Leu) | XTY | Glutamine (Gln) | CAJ |
| Isoleucine (Ile) | ATM | Asparagine (Asn) | AAK |
| Methionine (Met) | ATG | Lysine (Lys) | AAJ |
| Valine (Val) | GTL | Aspartic acid (Asp) | GAK |
| Serine (Ser) | QRS | Glutamic acid (Glu) | CAJ |
| Proline (Pro) | CCL | Cysteine (Cys) | TGK |
| Threonine (Thr) | ACL | Tryptophan (Trp) | TGG |
| Alanine (Ala) | GCL | Arginine (Arg) | WGZ |
| Tyrosine (Tyr) | TAK | Glycine (Gly) | GGL |
| Termination Signal | TAJ | | |
| Termination Signal | TGA | | |

Key: Each 3-letter deoxynucleotide triplet corresponds to a trinucleotide of mRNA, having a 5'-end on the left and a 3'-end on the right. All DNA sequences given herein are those of the strand whose sequence corresponds to the mRNA sequence, with thymine substituted for uracil. The letters stand for the purine or pyrimidine bases forming the deoxynucleotide sequence.

A = adenine

G = guanine

C = cytosine

T - thymine

X = T or C if Y is A or G

X = C if Y is C or T

Y = A, G, C or T if X is C

Y = A or G if X is T

W = C or A if Z is A or G

W = C if Z is C or T

Z = A, G, C or T if W is C

Z = A or G if W is A

QR = TC if S is A, G, C or T; alternatively QR = AG if S is T or C

J = A or G
K = T or C
L = A, T, C or G
M = A, C or T

The above shows that the novel amino acid sequences of the HTLV-III proteins of the subject invention can be prepared by nucleotide sequences other than those disclosed herein. Functionally equivalent nucleotide sequences encoding the novel amino acid sequences of these HTLV-III proteins, or fragments thereof having HTLV-III antigenic or immunogenic or therapeutic activity, can be prepared by known synthetic procedures. Accordingly, the subject invention includes such functionally equivalent nucleotide sequences.

Thus the scope of the subject invention includes not only the specific nucleotide sequences depicted herein, but also all equivalent nucleotide sequences coding for molecules with substantially the same HTLV-III antigenic or immunogenic or therapeutic activity.

Further, the scope of the subject invention is intended to cover not only the specific amino acid sequences disclosed, but also similar sequences coding for proteins or protein fragments having comparable ability to induce the formation of and/or bind to specific HTLV-III antibodies.

The term "equivalent" is being used in its ordinary patent usage here as denoting a nucleotide sequence which performs substantially as the nucleotide sequence identified herein to produce molecules with substantially the same HTLV-III antigenic or immunogenic or therapeutic activity in essentially the same kind of hosts. Within this definition are subfragments which have HTLV-III antigenic or immunogenic or therapeutic activity.

As disclosed above, it is well within the skill of those in the genetic engineering art to use the nucleotide sequences encoding HTLV-III antigenic or immunogenic or therapeutic activity of the subject invention to produce HTLV-III proteins via microbial processes. Fusing the sequences into an expression vector and transforming or transfecting into hosts, either eukaryotic (yeast or mammalian cells) or prokaryotic (bacterial cells), are standard procedures used in producing other well-known proteins, e.g., insulin, interferons, human growth hormone, IL-1, IL-2, and the like. Similar procedures, or obvious modifications thereof, can be employed to prepare HTLV-III proteins by microbial means or tissue-culture technology in accord with the subject invention.

The nucleotide sequences disclosed herein can be prepared by a "gene machine" by procedures well known in the art. This is possible because of the disclosure of the nucleotide sequence.

The restriction enzymes disclosed can be purchased from Bethesda Research Laboratories, Gaithersburg, MD, or New England Biolabs, Beverly, MA. The enzymes are used according to the instructions provided by the supplier.

The various methods employed in the preparation of the plasmids and transformation of host organisms are well known in the art. These procedures are all described in Maniatis, T., Fritsch, E.F., and Sambrook, J. (1982) Molecular Cloning: A Laboratory Manual, Cold Spring Harbor Laboratory, New York. Thus, it is within the skill of those in the genetic engineering art to extract DNA from microbial cells, perform restriction enzyme digestions, electrophorese DNA fragments, tail and anneal plasmid and insert DNA, ligate DNA, transform cells, e.g., E. coli cells, prepare plasmid DNA, electrophorese proteins, and sequence DNA.

Immunochemical assays employing the HTLV-III proteins of the invention can take a variety of forms. The preferred type is a solid phase immunometric assay. In assays of this type, an HTLV-III protein is immobilized on a solid phase to form an antigen-immunoadsorbent. The immunoadsorbent is incubated with the sample to be tested. After an appropriate incubation period, the immunoadsorbent is separated from the sample and labeled anti-(human IgG) antibody is used to detect human anti-HTLV-III antibody bound to the immunoadsorbent. The amount of label associated with the immunoadsorbent can be compared to positive and negative controls to assess the presence or absence of anti-HTLV-III antibody.

The immunoadsorbent can be prepared by adsorbing or coupling a purified HTLV-III protein to a solid phase. Various solid phases can be used, such as beads formed of glass, polystyrene, polypropylene, dextran or other material. Other suitable solid phases include tubes or plates formed from or coated with these materials.

The HTLV-III proteins can be either covalently or non-covalently bound to the solid phase by techniques such as covalent bonding via an amide or ester linkage or adsorption. After the HTLV-III protein is affixed to the solid phase, the solid phase can be post-coated with an animal protein, e.g., 3% fish gelatin. This provides a blocking protein which reduces nonspecific adsorption of protein to the immunoadsorbent surface.

The immunoadsorbent is then incubated with the sample to be tested for anti-HTLV-III antibody. In blood screening, blood plasma or serum is used. The plasma or serum is diluted with normal animal plasma

4

or serum. The diluent plasma or serum is derived from the same animal species that is the source of the anti-(human IgG) antibody. The preferred anti-(human IgG) antibody is goat anti-(human IgG) antibody. Thus, in the preferred format, the diluent would be goat serum or plasma.

The conditions of incubation, e.g., pH and temperature, and the duration of incubation are not crucial. These parameters can be optimized by routine experimentation. Generally, the incubation will be run for 1-2 hr at about 45°C in a buffer of pH 7-8.

After incubation, the immunoadsorbent and the sample are separated. Separation can be accomplished by any conventional separation technique such as sedimentation or centrifugation. The immunoadsorbent then may be washed free of sample to eliminate any interfering substances.

The immunoadsorbent is incubated with the labeled anti-(human IgG) antibody (tracer) to detect human antibody bound thereto. Generally the immunoadsorbent is incubated with a solution of the labeled anti-(human IgG) antibody which contains a small amount (about 1%) of the serum or plasma of the animal species which serves as the source of the anti-(human IgG) antibody. Anti-(human IgG) antibody can be obtained from any animal source. However, goat anti-(human IgG) antibody is preferred. The anti-(human IgG) antibody can be an antibody against the Fc fragment of human IgG, for example, goat anti-(human IgG) Fc antibody.

The anti-(human IgG)antibody or anti-(human IgG)Fc can be labeled with a radioactive material such as $^{125}$iodine; labeled with an optical label, such as a fluorescent material; or labeled with an enzyme such as horseradish peroxidase. The anti-human antibody can also be biotinylated and labeled avidin used to detect its binding to the immunoadsorbent.

After incubation with the labeled antibody, the immunoadsorbent is separated from the solution and the label associated with the immunoadsorbent is evaluated. Depending upon the choice of label, the evaluation can be done in a variety of ways. The label may be detected by a gamma counter if the label is a radioactive gamma emitter, or by a fluorimeter, if the label is a fluorescent material. In the case of an enzyme, label detection may be done colorimetrically employing a substrate for the enzyme.

The amount of label associated with the immunoadsorbent is compared with positive and negative controls in order to determine the presence of anti-HTLV-III antibody. The controls are generally run concomitantly with the sample to be tested. A positive control is a serum containing antibody against HTLV-III; a negative control is a serum from healthy individuals which does not contain antibody against HTLV-III.

For convenience and standardization, reagents for the performance of the immunometric assay can be assembled in assay kits. A kit for screening blood, for example, can include:

(a) an immunoadsorbent, e.g., a polystyrene bead coated with an HTLV-III protein;

(b) a diluent for the serum or plasma sample, e.g., normal goat serum or plasma;

(c) an anti-(human IgG) antibody, e.g., goat anti-(human IgG) antibody in buffered, aqueous solution containing about 1% goat serum or plasma;

(d) a positive control, e.g., serum containing antibody against at least one of the novel HTLV-III proteins; and

(e) a negative control, e.g., pooled sera from healthy individuals which does not contain antibody against at least one of the novel HTLV-III proteins.

If the label is an enzyme, an additional element of the kit can be the substrate for the enzyme.

Another type of assay for anti-HTLV-III antibody is an antigen sandwich assay. In this assay, a labeled HTLV-III protein is used in place of anti-(human IgG) antibody to detect anti-HTLV-III antibody bound to the immunoadsorbent. The assay is based in principle on the bivalency of antibody molecules. One binding site of the antibody binds the antigen affixed to the solid phase; the second is available for binding the labeled antigen. The assay procedure is essentially the same as described for the immunometric assay except that after incubation with the sample, the immunoadsorbent is incubated with a solution of labeled HTLV-III protein. HTLV-III proteins can be labeled with radioisotope, an enzyme, etc. for this type of assay.

In a third format, the bacterial protein, protein A, which binds the Fc segment of an IgG molecule without interfering with the antigen-antibody interaction can be used as the labeled tracer to detect anti-HTLV-antibody adsorbed to the immunoadsorbent. Protein A can be readily labeled with a radioisotope, enzyme or other detectable species.

Immunochemical assays employing an HTLV-III protein have several advantages over those employing a whole (or disrupted) virus. Assays based upon an HTLV-III protein will alleviate the concern over growing large quantities of infectious virus and the inherent variability associated with cell culturing and virus production. Further, the assay will help mitigate the real or perceived fear of contracting AIDS by technicians in hospitals, clinics and blood banks who perform the test.

Vaccines of the HTLV-III proteins, disclosed herein, and variants thereof having antigenic properties, can be prepared by procedures well known in the art. For example, such vaccines can be prepared as injectables, e.g., liquid solutions or suspensions. Solid forms for solution in, or suspension in, a liquid prior to injection also can be prepared. Optionally, the preparation also can be emulsified. The active antigenic ingredient or ingredients can be mixed with excipients which are pharmaceutically acceptable and compatible with the active ingredient. Examples of suitable excipients are water, saline, dextrose, glycerol, ethanol, or the like, and combinations thereof. In addition, if desired, the vaccine can contain minor amounts of auxiliary substances such as wetting or emulsifying agents, pH buffering agents, or adjuvants which enhance the effectiveness of the vaccine. The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers include, for example, polyalkalene glycols or triglycerides. Suppositories can be formed from mixtures containing the active ingredient in the range of about 0.5%, to about 10, preferably about 1 to about 2%. Oral formulations can include such normally employed excipients as, for example, pharmaceutical grades of manitol, lactose, starch, magnesium stearate, sodium saccharine, cellulose, magnesium carbonate and the like. These compositions can take the form of solutions, suspensions, tablets, pills, capsules, sustained release formulations or powders and contain from about 10% to about 95% of active ingredient, preferably from about 25% to about 70%.

The proteins can be formulated into the vaccine as neutral or salt forms. Pharmaceutically acceptable salts include the acid addition salts (formed with the free amino groups of the peptide) and which are formed with inorganic acids such as, for example, hydrochloric or phosphoric acids, or such organic acids as acetic, oxalic, tartaric, mandelic, and the like. Salts formed with the free carboxyl groups also can be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The vaccines are administered in a manner compatible with the dosage formulation, and in such amount as will be therapeutically effective and immunogenic. The quantity to be administered depends on the subject to be treated, capacity of the subject's immune system to synthesize antibodies, and the degree of protection desired. Precise amounts of active ingredient required to be administered depend on the judgment of the practitioner and are peculiar to each individual. However, suitable dosage ranges are of the order of about several hundred micrograms active ingredient per individual. Suitable regimes for initial administration and booster shots are also variable, but are typified by an initial administration followed in one or two week intervals by a subsequent injection or other administration.

HTLV-III is known to undergo amino acid sequence variation, particularly in the envelope gene (Starcich, B.R. [1986] Cell 45:637-648; Hahn, B.H. et al. [1986] Science 232:1548-1553). Over 100 variants have been analyzed by molecular cloning and restriction enzyme recognition analysis, and several of these have been analyzed by nucleotide sequencing. Some of these are the HTLV-III isolates known as RF (Popovic, M. et al. [1984] Science 224:497-500), WMJ-1 (Hahn, B.H. et al. [1986] Science 232:1548-1553), LAV (Wain-Hobson, S. et al. [1985] Cell 40:9-17), and ARV-2 (Sanchez-Pescador, R. et al. [1985] Science 227:484-492). Although the subject invention describes the sequence from one HTLV-III isolate, the appropriate envelope regions of any HTLV-III isolate can be produced using the procedures described herein for preparing R10, PB1, 590, and KH1. The HTLV-III proteins from different viral isolates can be used in vaccine preparations, as disclosed above, to protect against infections by different HTLV-III isolates. Further, a vaccine preparation can be made using more than one recombinant antigenic protein from more than one HTLV-III isolate, to provide immunity and thus give better protection against AIDS.

Following are examples which illustrate the process of the invention, including the best mode. These examples should not be construed as limiting. All solvent mixture proportions are by volume unless otherwise noted.

Example I--Construction of plasmid pREV2.2

The pREV2.2 plasmid expression vector was constructed from plasmid pBG1. Plasmid pBG1 can be isolated from its E. coli host by well known procedures, e.g., using cleared lysate-isopycnic density gradient procedures, and the like. Like pBG1, pREV2.2 expresses inserted genes behind the E. coli promoter. The differences between pBG1 and pREV2.2 are the following:

1. pREV2.2 lacks a functional replication of plasmid (rop) protein.

2. pREV2.2 has the trpA transcription terminator inserted into the AatII site. This sequence insures transcription termination of over-expressed genes.

3. pREV2.2 has genes to provide resistance to ampicillin and chloramphenicol, whereas pBG1 provides resistance only to ampicillin.

6

4. pREV2.2 contains a sequence encoding sites for several restriction endonucleases.

The following procedures, shown in Figure 1 of the drawings, were used to make each of the four changes listed above:

1a. 5 μg of plasmid pBG1 was restricted with NdeI which gives two fragments of approximately 2160 and 3440 base pairs.

1b. 0.1 μg of DNA from the digestion mixture, after inactivation of the NdeI, was treated with T4 DNA ligase under conditions that favor intramolecular ligation (200 μl reaction volume using standard T4 ligase reaction conditions [New England Biolabs, Beverly, MA]). Intramolecular ligation of the 3440 base pair fragment gave an ampicillin resistant plasmid. The ligation mixture was transformed into the recipient strain E. coli JM103 (available from New England Biolabs) and ampicillin resistant clones were selected by standard procedures.

1c. The product plasmid, pBG1ΔN, where the 2160 base pair NdeI fragment is deleted from pBG1, was selected by preparing plasmid from ampicillin resistant clones and determining the restriction digestion patterns with NdeI and SalI (product fragments approximately 1790 and 1650). This deletion inactivates the rop gene that controls plasmid replication.

2a. 5 μg Of pBG1ΔN was then digested with EcoRI and BclI and the larger fragment, approximately 2455 base pairs, was isolated.

2b. A synthetic double stranded fragment was prepared by the procedure of Itakura et al. (Itakura, K., Rossi, J.J. and Wallace, R.B. [1984] Ann. Rev. Biochem. 53:323-356, and references therein) with the structure shown in Table 1. This fragment has BclI and EcoRI sticky ends and contains recognition sequences for several restriction endonucleases.

2c. 0.1 μg of the 2455 base pair EcoRI-BclI fragment and 0.01 μg of the synthetic fragment were joined with T4 DNA ligase and competent cells of strain JM103 were transformed. Cells harboring the recombinant plasmid, where the synthetic fragment was inserted into pBG1ΔN between the BclI and EcoRI sites, were selected by digestion of the plasmid with HpaI and EcoRI. The diagnostic fragment sizes are approximately 2355 and 200 base pairs. This plasmid is called pREV1.

2d. 5 μg of pREV1 were digested with AatII, which cleaves uniquely.

2e. The double stranded fragment shown in Table 2 was synthesized. This fragment has AatII sticky ends and contains the trpA transcription termination sequence.

2f. 0.1 μg of AatII digested pREV1 was ligated with 0.01 μg of the synthetic fragment in a volume of 20 μl using T4 DNA ligase.

2g. Cells of strain JM103, made competent, were transformed and ampicillin resistant clones selected.

2h. Using a KpnI, EcoRI double restriction digest of plasmid isolated from selected colonies, a cell containing the correct construction was isolated. The sizes of the KpnI, EcoRI generated fragments are approximately 2475 and 80 base pairs. This plasmid is called pREV1TT and contains the trpA transcription terminator.

3a. 5 μg of pREV1TT, prepared as disclosed above (by standard methods) was cleaved with NdeI and XmnI and the approximately 850 base pair fragment was isolated.

3b. 5 μg of plasmid pBR325 (BRL, Gaithersburg, MD), which contains the genes conferring resistance to chloramphenicol as well as to ampicillin and tetracycline, was cleaved with BclI and the ends blunted with Klenow polymerase and deoxynucleotides. After inactivating the enzyme, the mixture was treated with NdeI and the approximately 3185 base pair fragment was isolated. This fragment contains the genes for chloramphenicol and ampicillin resistance and the origin of replication.

3c. 0.1 μg of the NdeI-XmnI fragment from pREV1TT and the NdeI-BclI fragment from pBR325 were ligated in 20 μl with T4 DNA ligase and the mixture used to transform competent cells of strain JM103. Cells resistant to both ampicillin and chloramphenicol were selected.

3d. Using an EcoRI and NdeI double digest of plasmid from selected clones, a plasmid was selected giving fragment sizes of approximately 2480, 1145, and 410 base pairs. This is called plasmid pREV1TT/ch1 and has genes for resistance to both ampicillin and chloramphenicol.

4a. A double stranded fragment shown in Table 3 was synthesized. This fragment, with a blunt end and an SstI sticky end, contains recognition sequences for several restriction enzyme sites.

4b. 5 μg of pREV1TT/ch1 was cleaved with NruI (which cleaves about 20 nucleotides from the BclI site) and SstI (which cleaves within the multiple cloning site). The larger fragment, approximately 3990 base pairs, was isolated from an agarose gel.

4c. 0.1 μg of the NruI-SstI fragment from pREV1TT/ch1 and 0.01 μg of the synthetic fragment were treated with T4 DNA ligase in a volume of 20 μl.

4d. This mixture was transformed into strain JM103 and ampicillin resistant clones were selected.

4e. Plasmid was purified from several clones and screened by digestion with MluI or ClaI. Recombinant

clones with the new multiple cloning site will give one fragment when digested with either of these enzymes, because each cleaves the plasmid once.

4f. The sequence of the multiple cloning site was verified. This was done by restricting the plasmid with HpaI and PvuII and isolating the 1395 base pair fragment, cloning it into the SmaI site of mp18 and sequencing it by dideoxynucleotide sequencing using standard methods.

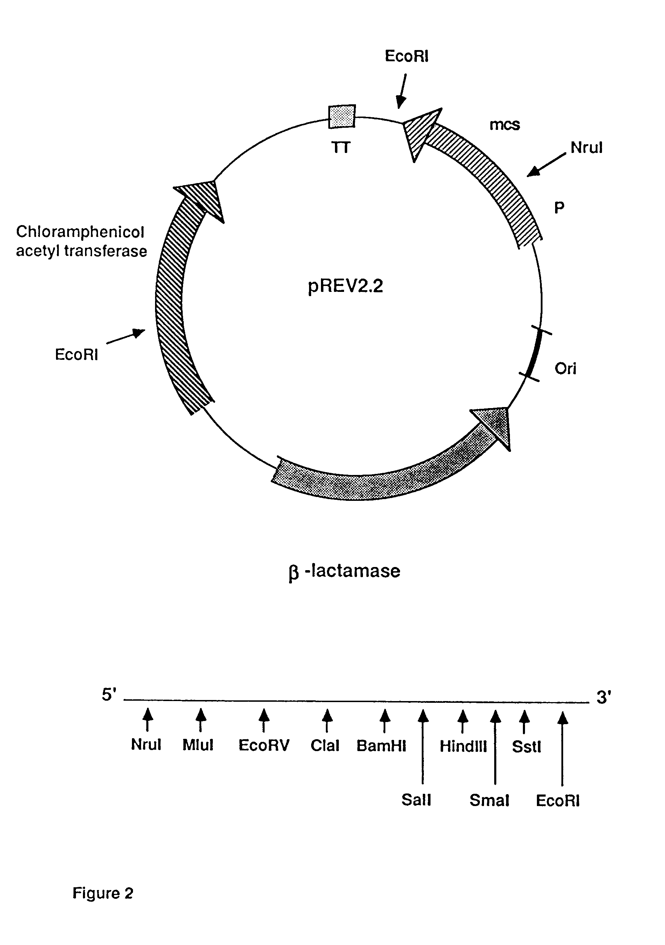

4g. This plasmid, called pREV2.2, is shown in Figure 2 of the drawings.

Example 4--Construction of and expression from plasmid pPB1

Plasmid pPB1, which contains approximately 540 base pairs of DNA encoding essentially the HTLV-III env gene from the PvuII site to the BglII site, and from which is synthesised an approximately 26 kD fusion protein containing this portion of the gp120 envelope protein can be constructed as follows:

1. Synthesising the DNA with the sequence shown in Table 4: This DNA fragment can be synthesised by standard methods and encodes a portion of gp120. It has a blunt end on the 5' end and an end which will ligate with a BamHI overhang on the 3' end.

2. Restricting 5 $\mu$g plasmid pREV2.2 with EcoRV and BamHI and isolating the large fragment, approximately 4 kD, from an agarose gel.

3. Ligating 0.1 $\mu$g of the fragment in Table 4 with 0.1 $\mu$g of the pREV2.2 fragment in a volume of 20 $\mu$l using T4 DNA ligase, transforming the ligation mixture into competent cell strain SG20251, and selecting ampicillin resistant transformants.

4. Using the AhaIII restriction pattern of purified plasmid, selecting cells harboring the recombinant plasmid with the synthesized fragment in the orientation whereby the fragment blunt end ligated to the REV2.2 EcoRV end and the BamHI overhanging ends ligated together. AhaIII digestion of the proper plasmid gives fragment lengths of approximately 1210, 1020, 750, 690, 500, 340, and 20 base pairs. When the strain harboring this recombinant plasmid is grown in 2% medium containing 50 $\mu$g/ml ampicillin and the total complement of cellular proteins electrophoresed on an SDS-polyacrylamide gel, a protein of approximately 26 kD can be visualized by either coomassie blue staining or by western blot analysis using as probe selected sera from AIDS, ARC, or HTLV-III infected individuals.

Example 5--Purification of recombinant protein containing HTLV-III envelope sequences from plasmid pPB1

1. Growth of cells:

Cells were grown in a 10 liter volume in a Chemap fermentor in 2% medium. Fermentation temperature was 37°C, the pH was 6.8, and air was provided at 1 vvm. Plasmid selection was provided by 50 ug/ml ampicillin and 20 ug/ml chloramphenicol. Typical cell yield (wet weight) was 30 g/l.

2. Cell Lysis:

50 g, wet cell weight, of E. coli containing the recombinant HTLV-III envelope fusion protein were resuspended in a final volume of 100 ml in 50 mM Tris-Cl pH 8.0, 5 mM KEDTA, 5 mM DTT, 15 mM $\beta$-mercaptoethanol, 0.5% Triton X-100, and 5 mM PMSF. 300 mg lysozyme was added and the suspension incubated for 30 min at room temperature.

This material was lysed using a BEAD-BEATER™ (Biospec Products, Bartlesville, OK) containing an equal volume of 0.1-0.15 $\mu$m glass beads. The lysis was done for 6 min at room temperature in 1 min intervals. The liquid was separated from the beads and centrifuged for 2.5 hr at 20,000 xg. The supernatant was removed and the pellet was resuspended in 100 ml 6 M guanidine-hydrochloride, 20 mM Tris-Cl pH 8.0, 5 mM DTT, 15 mM $\beta$-mercaptoethanol, 5 mM PMSF, and 1 mM KEDTA. The pellet was solubilized using a polytron homogenizer and centrifuged at 20,000 xg for 2 hr.

The supernate (90 ml) was dialysed against 4 liters of 8 M urea, 20 mM Tris-Cl, pH 8.0, 1 mM EDTA, and 15 mM $\beta$-mercaptoethanol. Dialysis was done each time for 8 hr or longer with three changes of buffer. Spectraphor dialysis tubing (S/P, McGraw Park, IL) with a 3.5 kD MW cut-off was used. 3. CM chromatography

The dialysate was loaded onto a 550 ml column (5 cm x 28 cm) packed with CM Fast Flow Sepharose (Pharmacia) equilibrated in 8 M urea, 10 mM potassium phosphate pH 7.0, 15 mM $\beta$-mercaptoethanol, and 1 mM KEDTA at room temperature. The column was washed with 2 liters equilibration buffer, and the protein eluted with a 5.0 liter linear gradient from 0-0.4 M NaCl. The HTLV-III protein (26 kD) eluted at approximately 0.2 M NaCl as assayed by SDS-polyacrylamide gel electrophoresis.

## Table 1

5' GATCAAGCTTCTGCAGTCGACGCATGCGGATCCGGTACCCGGGAGCTCG 3'
    TTCGAAGACGTCAGCTGCGTACGCCTAGGCCATGGGCCCTCGAGCTTAA

## Table 2

5'        CGGTACCAGCCCGCCTAATGAGCGGGCTTTTTTTTTGACGT        3'
     TGCAGCCATGGTCGGGCGGATTACTCGCCCGAAAAAAAAC

## Table 3

MluI      EcoRV  ClaI BamHI  SalI HindIII SmaI

CGAACGCGTGGCCGATATCATCGATGGATCCGTCGACAAGCTTCCCGGGAGCT
GCTTGCGCACCGGCTATAGTAGCTACCTAGGCAGCTGTTCGAAGGGCCC

9

## Table 4

```
5'   CTGAACCAATCTGTAGAAATTAATTGTACAAGACCCAAC
     GACTTGGTTAGACATCTTTAATTAACATGTTCTGGGTTG

AACAATACAAGAAAAAGTATCCGTATCCAGAGAGGACCAGGGAGAGCATTTGTT
TTGTTATGTTCTTTTTCATAGGCATAGGTCTCTCCTGGTCCCTCTCGTAAACAA

ACAATAGGAAAAATAGGAAATATGAGACAAGCACATTGTAACATTAGTAGAGCA
TGTTATCCTTTTTATCCTTTATACTCTGTTCGTGTAACATTGTAATCATCTCGT

AAATGGAATAACACTTTAAAACAGATAGATAGCAAATTAAGAGAACAATTTGGA
TTTACCTTATTGTGAAATTTTGTCTATCTATCGTTTAATTCTCTTGTTAAACCT

AATAATAAAACAATAATCTTTAAGCAGTCCTCAGGAGGGGACCCAGAAATTGTA
TTATTATTTTGTTATTAGAAATTCGTCAGGAGTCCTCCCCTGGGTCTTTAACAT

ACGCACAGTTTTAATTGTGGAGGGGAATTTTTCTACTGTAATTCAACACAACTG
TGCGTGTCAAAATTAACACCTCCCCTTAAAAAGATGACATTAAGTTGTGTTGAC

TTTAATAGTACTTGGTTTAATAGTACTTGGAGTACTAAAGGGTCAAATAACACT
AAATTATCATGAACCAAATTATCATGAACCTCATGATTTCCCAGTTTATTGTGA

GAAGGAAGTGACACAATCACCCTCCCATGCAGAATAAAACAAATTATAAACATG
CTTCCTTCACTGTGTTAGTGGGAGGGTACGTCTTATTTTGTTTAATATTTGTAC

TGGCAGGAAGTAGGAAAAGCAATGTATGCCCCTCCCATCAGTGGACAAATTAGA
ACCGTCCTTCATCCTTTTCGTTACATACGGGGAGGGTAGTCACCTGTTTAATCT

TGTTCATCAAATATTACAGGGCTGCTATTAACAAGAGATGGTGGTAATAGCAAC
ACAAGTAGTTTATAATGTCCCGACGATAATTGTTCTCTACCACCATTATCGTTG

AATGAGTCCGA              3'
TTACTCAGGCTCTAG
```

## Table 5
## Amino acid sequence of fusion protein PB1

MetLeuArg

ProValGluThrProThrArgGluIleLysLysLeuAspGlyLeuTrpAlaPhe

SerLeuAspArgGluArgValAlaAspLeuAsnGlnSerValGluIleAsnCys

ThrArgProAsnAsnAsnThrArgLysSerIleArgIleGlnArgGlyProGly

ArgAlaPheValThrIleGlyLysIleGlyAsnMetArgGlnAlaHisCysAsn

IleSerArgAlaLysTrpAsnAsnThrLeuLysGlnIleAspSerLysLeuArg

GluGlnPheGlyAsnAsnLysThrIleIlePheLysGlnSerSerGlyGlyAsp

ProGluIleValThrHisSerPheAsnCysGlyGlyGluPhePheTyrCysAsn

SerThrGlnLeuPheAsnSerThrTrpPheAsnSerThrTrpSerThrLysGly

SerAsnAsnThrGluGlySerAspThrIleThrLeuProCysArgIleLysGln

IleIleAsnMetTrpGlnGluValGlyLysAlaMetTyrAlaProProIleSer

GlyGlnIleArgCysSerSerAsnIleThrGlyLeuLeuLeuThrArgAspGly

GlyAsnSerAsnAsnGluSerGluIleArgArgGlnAlaSerArgGluLeuGlu

PheLeuLysThrLysGlyProArgAspThrProIlePheIleGly

Table 5A

Nucleotide sequence encoding fusion protein PB1

```
ATGTTACGTCCTGTAGAAACCCCAACCCGTGAAATCAAAAAACTCGACGGCCTG
TACAATGCAGGACATCTTTGGGGTTGGGCACTTTAGTTTTTTGAGCTGCCGGAC

TGGGCATTCAGTCTGGATCGCGAACGCGTGGCCGATCTGAACCAATCTGTAGAA
ACCCGTAAGTCAGACCTAGCGCTTGCGCACCGGCTAGACTTGGTTAGACATCTT

ATTAATTGTACAAGACCCAACAACAATACAAGAAAAAGTATCCGTATCCAGAGA
TAATTAACATGTTCTGGGTTGTTGTTATGTTCTTTTTCATAGGCATAGGTCTCT

GGACCAGGGAGAGCATTTGTTACAATAGGAAAAATAGGAAATATGAGACAAGCA
CCTAATCCCTCTCGTAAACAATGTTATCCTTTTTATCCTTTATACTCTGTTCGT

CATTGTAACATTAGTAGAGCAAAATGGAATAACACTTTAAAACAGATAGATAGC
GTAACATTGTAATCATCTCGTTTTACCTTATTGTGAAATTTTGTCTATCTATCG

AAATTAAGAGAACAATTTGGAAATAATAAAACAATAATCTTTAAGCAGTCCTCA
TTTAATTCTCTTGTTAAACCTTTATTATTTTGTTATTAGAAATTCGTCAGGAGT

GGAGGGGACCCAGAAATTGTAACGCACAGTTTTAATTGTGGAGGGGAATTTTTC
CCTCCCCTGGGTCTTTAACATTGCGTGTCAAAATTAACACCTCCCCTTAAAAAG

TACTGTAATTCAACACAACTGTTTAATAGTACTTGGTTTAATAGTACTTGGAGT
ATGACATTAAGTTGTGTTGACAAATTATCATGAACCAAATTATCATGAACCTCA

ACTAAAGGGTCAAATAACACTGAAGGAAGTGACACAATCACCCTCCCATGCAGA
TGATTTCCCAGTTTATTGTGACTTCCTTCACTGTGTTAGTGGGAGGGTACGTCT

ATAAAACAAATTATAAACATGTGGCAGGAAGTAGGAAAAGCAATGTATGCCCCT
TATTTTGTTTAATATTTGTACACCGTCCTTCATCCTTTTCGTTACATACGGGGA

CCCATCAGTGGACAAATTAGATGTTCATCAAATATTACAGGGCTGCTATTAACA
GGGTAGTCACCTGTTTAATCTACAAGTAGTTTATAATGTCCCGACGATAATTGT

AGAGATGGTGGTAATAGCAACAATGAGTCCGAGATCCGTCGACAAGCTTCCCGG
TCTCTACCACCATTATCGTTGTTACTCAGGCTCTAGGCAGCTGTTCGAAGGGCC

GAGCTCGAATTCTTGAAGACGAAAGGGCCTCGTGATACTCCTATTTTTATAGGT
CTCGAGCTTAAGAACTTCTGCTTTCCCGGAGCACTATGCGGATAAAAATATCCA
```

**Claims**

1. A protein having the amino-acid sequence shown in Table 5, or an immunologically-active fragment thereof.

2. A protein according to claim 2, for therapeutic use.

3. A vaccine composition which comprises a protein having the amino-acid sequence shown in Table 5, in a pharmacologically-acceptable vehicle.

4. DNA having the nucleotide sequence shown in Table 5A, or an equivalent nucleotide sequence containing bases whose translated region codes for a protein having the amino-acid sequence shown in Table 5.

5. A recombinant DNA transfer vector comprising DNA having all or part of the nucleotide sequence shown in Table 5A or an equivalent nucleotide sequence containing bases whose translated region codes for the protein having the amino-acid sequence shown in Table 5.

6. Plasmid pPB1, as available in E. coli SG20251, NRRL B-18092.

7. A host transformed by the transfer vector of claim 5.

8. An immunochemical assay for detecting or quantifying antibody against HIV in a fluid, which comprises employing a protein having the amino-acid sequence shown in Table 5.

9. An immunoadsorbent suitable for use in a solid phase immunochemical assay for antibody against HIV, which comprises a solid phase to which is affixed a protein having the amino-acid sequence shown in Table 5.

10. An immunoadsorbent according to claim 9, wherein the solid phase is a glass or plastic bead, a well of a microtiter plate or a test tube.

11. An immunoadsorbent according to claim 9 or claim 10, which additionally comprises a post-coat of animal protein.

12. A kit suitable for use in detecting antibody against HIV in a biological fluid, which comprises:
    (a) an immunoadsorbent according to any of claims 9 to 11;
    (b) labelled HIV antibody; and
    (c) means for detecting the label associated with the immunoadsorbent.

13. A kit according to claim 12, wherein the label is anti-(human IgG) antibody.

14. A method of detecting antibody against HIV in a biological fluid, which comprises the steps of:
    (a) incubating an immunoadsorbent according to any of claims 9 to 11 with a sample of the biological fluid, under conditions which allow anti-HIV antibody in the sample to bind to the immunoadsorbent;
    (b) separating the immunoadsorbent from the sample; and
    (c) determining if antibody has bound to the immunoadsorbent as an indication of anti-HIV in the sample.

15. A method according to claim 14, wherein step (c) comprises incubating the immunoadsorbent with a labelled antibody against antigen of the species from which the biological fluid is derived; separating the immunoadsorbent from the labelled antibody; and detecting the label associated with the immunoadsorbent.

16. A method according to claim 14, wherein step (c) comprises incubating the immunoadsorbent with labelled protein having the amino-acid sequence shown in Table 5; separating the immunoadsorbent from the labelled protein; and detecting the label associated with the immunoadsorbent.

17. A method according to claim 14, wherein step (c) comprises incubating the immunoadsorbent with labelled protein A; separating the immunoadsorbent from the labelled protein A; and detecting the label associated with the immunoadsorbent.

18. A method of detecting antibody against HIV in a human serum or plasma sample, which comprises the steps of:
    (a) incubating a bead of an immunoadsorbent according to any of claims 9 to 11 with the serum or plasma sample under conditions which allow anti-HIV antibody in the sample to bind to the immunoadsorbent;
    (b) separating the immunoadsorbent and the sample;
    (c) incubating the immunoadsorbent with a labelled anti-(human IgG) antibody under conditions which allow the anti-(human IgG) antibody to bind human anti-HIV antibody bound to the immunoadsorbent;

13

(d) separating the immunoadsorbent from the unbound anti-(human IgG) antibody; and

(e) evaluating the label associated with the immunoadsorbent as an indication of the presence of antibody against HIV in the sample.

19. A method according to claim 18, wherein the anti-(human IgG) antibody is an animal antibody and the serum or plasma sample is diluted with normal serum of an animal of the same species.

20. A method according to claim 19, wherein the anti-(human IgG) antibody is a goat antibody and the serum or plasma sample is diluted with normal goat serum.

21. A method according to any of claims 18 to 20, wherein the anti-(human IgG) antibody is labelled with a radioisotope, an enzyme or a fluorescent compound.

Figure 1A

Figure 18

β -lactamase

Figure 2

Fig. 3